# EUROPEAN PATENT SPECIFICATION

(11) **EP 3 785 681 B1**
(45) Date of publication and mention of the grant of the patent: **19.10.2022**
(21) Application number: 18887227.9
(22) Date of filing: 10.11.2018
(51) Int. Cl.: A61F 5/445, A61F 5/44, A61F 5/448

(54) **OSTOMY WASTE MATTER COLLECTION DEVICE**
VORRICHTUNG ZUR SAMMLUNG VON STOMAABFALL
DISPOSITIF DE COLLECTE DES DÉCHETS DE STOMIE

(30) Priority: 10.07.2018 CN 201810752868
(43) Date of publication of application: 03.03.2021
(73) Proprietor: ZHENDE MEDICAL CO., LTD., Shaoxing, Zhejiang 312035 (CN)
(72) Inventor: HU, Xiuyuan, Shaoxing, Zhejiang 312035 (CN); ZHANG, Rui, Shaoxing, Zhejiang 312035 (CN); LI, Zheng, Shaoxing, Zhejiang 312035 (CN)
(74) Representative: Loo, Chi Ching
(86) International application number: PCT/CN2018/114929
(87) International publication number: WO 2020/010766

(56) References cited:
- CN-A- 102 481 201
- CN-A- 102 481 201
- CN-A- 104 434 372
- CN-Y- 2 683 075
- US-A1- 2003 220 621
- US-A1- 2013 197 458
- US-A1- 2013 197 458

## Description

### FIELD OF THE INVENTION

This invention relates to a field of a medical product and, more particularly, to an ostomy waste matter collection device, according to claim 1.

### BACKGROUND OF THE INVENTION

The ostomy is generally aimed at lesions of the rectal and the bladder (such as rectal cancer, bladder cancer, intestinal obstruction, etc.). In order to save the patient's life, the doctor surgically removes the lesions. For example, the rectum and anal canal are removed for the rectal cancer, and the bladder is removed for the bladder cancer, then an opening is made on the left or right side of the patient's abdomen. The stool or urine is involuntarily excreted through the ostomy. This kind of patients will need to attach a pouch to the ostomy to place the waste matter after leaving the hospital, and this pouch is usually called an ostomy pouch. Currently, the ostomy pouch is usually adhered to the skin surrounding the ostomy through a hydrocolloid flange (or other sticky flanges). The incidence of problems of the skin surrounding the ostomy is as high as 60%, which seriously affects the quality of life of the person with the ostomy. These problems of the skin include irritant dermatitis and allergic dermatitis caused by the sticky flange, mechanical damage caused by peeling the sticky flange from the skin, and skin impregnation and fungal infection caused by leakage of the waste matter. A hole with the same size as the ostomy is cut in the center of the existing ostomy pouch flange on the market, and a gap between the opening of the flange and the ostomoy is coated with a leakage-proof paste. The main problem is that the waste matter is exposed on the exposed ostomy, and the waste matter leaks inward along the exposed ostomy and reaches the skin surrounding the ostomy, thereby causing a fester of the skin surrounding the ostomy. This invention will solve these problems. US2013/197458 discloses an ostomy waste matter collection device according to the preamble of claim 1.

### BRIEF SUMMARY OF THE INVENTION

To solve deficiencies in the prior art, this invention provides an ostomy waste matter collection device as claimed in claim 1. The second sealing ring and the sealing member cooperate to improve the sealing property, thereby avoiding leakage of the waste matter; by disposing the discharge valve, the discharge of the waste matter in the intestine can be controlled, such that the patient with the ostomy can regularly discharge the waste matter; and by disposing the ostomy protection assembly, the waste matter in the intestine can be effectively controlled and prevented from leaking to the skin surrounding the ostomy, thereby avoiding ulceration of the skin and ensuring health of the patient. The elastic bag preferably is coated with a lubricating and sealing reagent.

An absorbent material is preferably disposed between the turning horn mouth and the ostomy exposed portion.

The ostomy waste matter collection device may further include an extension tube connected with the ostomy connection tube.

The ostomy waste matter collection device may further include a collection pocket connected with the extension tube, and one end of the collection pocket may be provided with a discharge controlling assembly.

The discharge controlling assembly may include: a sealing pocket extended from the collection pocket, a self-sealing strip fixed at the sealing pocket, a soft magnetic strip disposed at a lower end of the self-sealing strip, and a handle fixed at the sealing pocket and located at a lower end of the soft magnetic strip.

Benefits of this invention are as follow.

In this invention, after the second sealing ring enters the ostomy, the second sealing ring opens the intestinal wall and is tightly close to the abdominal wall, and the second sealing ring and the elastic ostomy connection tube cooperate to form a first section sealing; the elastic bag is inflated through the second inflatable connection tube, and the elastic bag is tightly close to the intestinal wall forming a second section sealing; and the first section sealing and the second section sealing cooperate to prevent the waste matter in the intestine from leaking along the intestinal wall.

The discharge valve is the multi-cavity inflatable bag, the multi-cavity inflatable bag is inflated, and bags expand inward to close the cavity of the ostomy connection tube. In this way, the discharge of the waste matter in the intestine can be controlled, such that the patient with the ostomy can regularly discharge the waste matter.

By disposing the ostomy protection assembly, the structure that is turned outward along the ostomy can protect the ostomy intestinal tissue exposed outside the abdominal wall, and the waste matter in the intestine can be effectively controlled and prevented from leaking to the skin surrounding the ostomy, thereby avoiding ulceration of the skin and ensuring health of the patient.

The second sealing ring and the sealing member cooperate, such that the ostomy connection tube can be fixed in the ostomy stably, and the sticky flange is avoided to be adhered around the ostomy, thereby improving comfort and eliminating skin problems brought by the sticky flange.

### BRIEF DESCRIPTION OF THE DRAWINGS

FIG. 1 is a structural schematic diagram of one embodiment according to this invention;
FIG. 2 is a structural schematic diagram of one embodiment when a discharge valve is not inflated according to this invention;
FIG. 3 is a structural schematic diagram of one embodiment when the discharge valve is inflated according to this invention;
FIG. 4 is a structural schematic diagram of one embodiment during usage according to this invention;
FIG. 5 is a schematic diagram of an ostomy; and
FIG. 6 is a structural schematic diagram of one embodiment of a collection pocket according to this invention.

Figure reference numerals:
1 ostomy connection tube, 2 first sealing ring, 3 elastic bag, 4 second inflatable connection tube, 5 multi-cavity inflatable bag, 6 first inflatable connection tube, 7 second sealing ring, 8 absorbent material, 9 ostomy exposed portion, 10 abdominal wall, 11intraabdominal intestinal tract, 12 turning horn mouth, 13 extension tube, 14 collection pocket, 15 self-sealing strip, 16 sealing pocket, and 17 handle.

### DETAILED DESCRIPTION OF THE INVENTION

This invention is described in detail below in combination with the accompanying drawings and specific embodiments.

An ostomy waste matter collection device includes: an ostomy connection tube 1 fixed in an ostomy, a discharge valve disposed in the ostomy connection tube 1 and used for controlling the discharge of the waste matter, a sealing member disposed between the ostomy connection tube 1 and the abdominal wall for sealing, a turning outward line disposed between the discharge valve and the sealing member, and an ostomy protection assembly covering the ostomy exposed outside the abdominal wall and being connected with the ostomy connection tube 1. As a preferred way, the ostomy connection tube 1 is tubular taking an elastic film as the wall.

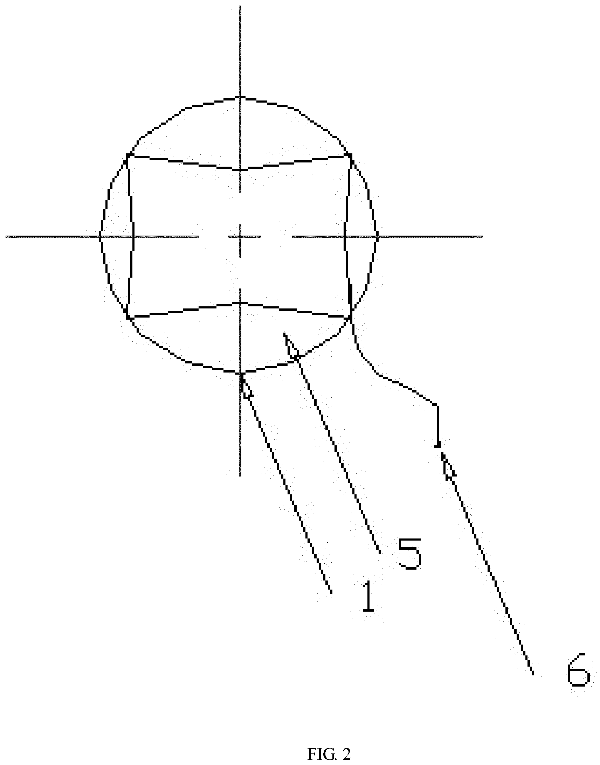

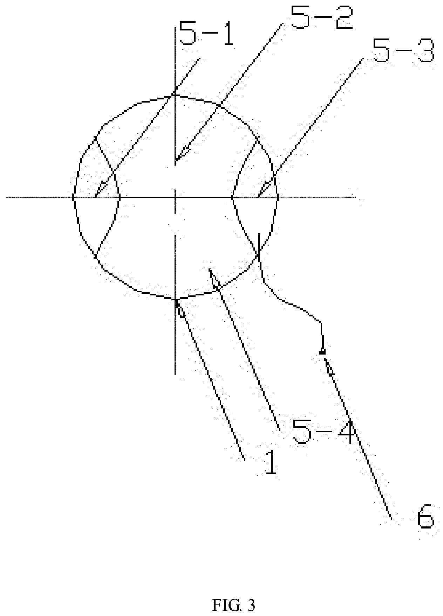

The discharge valve includes a multi-cavity inflatable bag 5 disposed in the ostomy connection tube 1 and a first inflatable connection tube 6 connected with the multi-cavity inflatable bag. FIG. 2 shows an opening state when the multi-cavity inflatable bag 5 is not inflated. There are four cavities in the circle, and blanks around the center of the circle are waste matter passages. FIG. 3 shows a closed state when the multi-cavity inflatable bag 5 is inflated. There are four cavity bags 5-1, 5-2, 5-3, and 5-4 in the circle, the four cavity bags expand inward after being inflated, and the water matter passages are closed.

The sealing member includes an annular elastic bag 3 disposed at the ostomy connection tube 1 and a second inflatable connection tube 4 connected with the elastic bag 3. In order to improve the sealing and comfort between the elastic bag 3 and the abdominal wall, the elastic bag 3 is coated with a lubricating and sealing reagent; as an embodiment, the lubricating and sealing reagent includes Vaseline or gel.

The ostomy protection assembly includes a turning horn mouth 12 extended at the ostomy connection tube 1 and used for covering an ostomy exposed portion, and a first sealing ring 2 disposed at ends of the turning horn mouth 12. It should be noted that in order to enable the ostomy connection tube 1 to be fixed in the ostomy in a sealing way, the diameter of the first sealing ring 2 is larger than the size of the ostomy. An absorbent material 8 is disposed between the turning horn mouth 12 and the ostomy exposed portion. It should be noted that the absorbent material adopted in this invention absorbs liquid of which the weight is more than 15 times heavier than the weight of the absorbent material. As an embodiment, the absorbent material 8 includes a PU foam, a superabsorbent nonwoven fabric (SAP), a hydrophilic fiber nonwoven fabric gelled after absorbing liquid, and the like. A second sealing ring 7 fixed in the ostomy is disposed at the turning outward line.

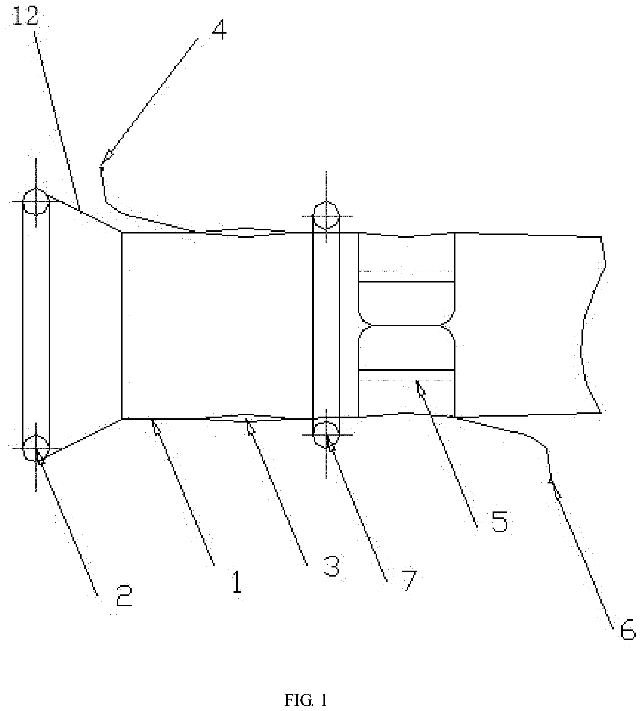

As shown in FIG. 1, the ostomy connection tube 1 is an elastic tube taking the elastic film as the wall, and one side of the tube is horn-shaped, that is, the turning horn mouth 12. Ends of the turning horn mouth 12 has a first sealing ring 2 made of the same elastic film as the tube, as a preferred way, the first sealing ring 2 is a first elastic O-shaped ring. The other end of the ostomy connection tube 1 is connected with a collection pocket 14, and the ostomy connection tube 1 can be connected with the collection pocket 14 as a whole or as two separate parts. There is an elastic bag 3 full of gas (liquid) 2-5 cm away from one side of the turning horn mouth 12 with the smaller diameter, and a second inflatable connection tube 4 of the elastic bag 3 is above the outer side of the ostomy connection tube 1. On the right side of the elastic bag 3, the multi-cavity inflatable bag 5 is disposed inside the ostomy connection tube 1, and the number of cavities of the multi-cavity inflatable bag 5 may be 2-10, preferably 4. The first inflatable connection tube 6 of the multi-cavity inflatable bag 5 is below the outer side of the elastic tube, and a second sealing ring 7 is disposed in the middle of the elastic bag 3 and the multi-cavity inflatable bag 5. As a preferred way, the second sealing ring 7 is a second elastic O-shaped ring. As an embodiment, the second elastic O-shaped ring can be connected with the ostomy connection tube 1 as a whole or as two separate parts.

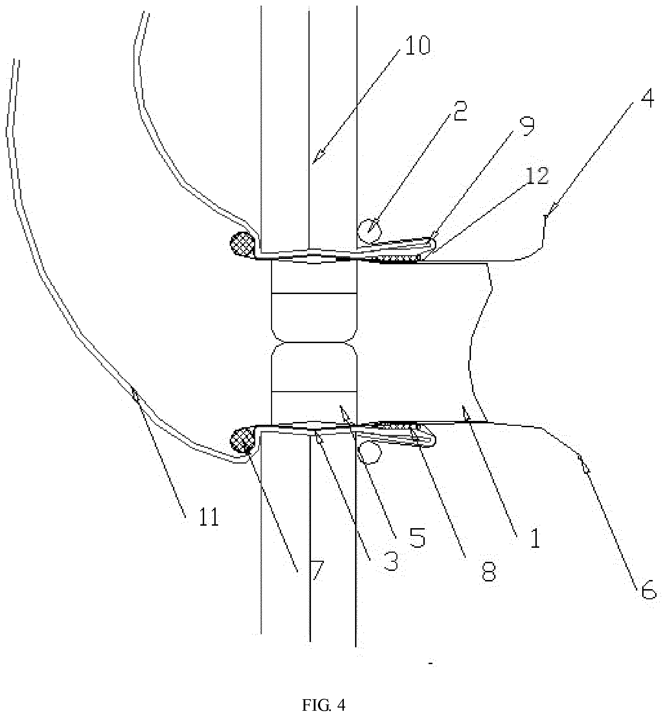

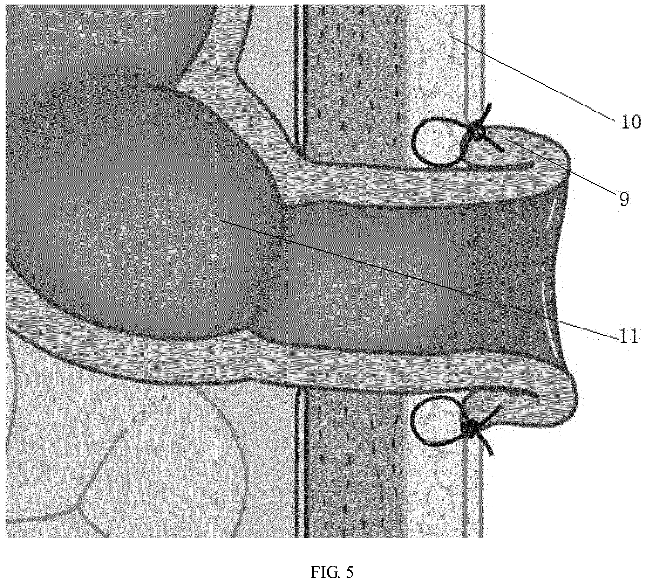

As shown in FIG. 4, the process of use is as follow. During usage, the turning outward line is taken as the center line, and the turning horn mouth 12 with the first sealing ring 2 is turned outward till the elastic bag 3 is aligned with or close to the multi-cavity inflatable bag 5. The outer wall of the elastic bag 3 is coated with the vaseline or gel for lubricating and sealing, and a layer of an annular superabsorbent material 8 is disposed at the right side of the outer side of the elastic bag 3. Then the second sealing ring 7 and the ostomy connection tube 1 are put inside the ostomy together, as shown in FIG. 5, 9 is the ostomy exposed portion, 10 is the abdominal wall, and 11 is an intraabdominal intestinal tract. After the second sealing ring 7 enters the ostomy, the second sealing ring 7 opens the intestinal wall and is tightly close to the abdominal wall, and the second sealing ring 7 and the elastic ostomy connection tube 1 cooperate to form a first section sealing; the elastic bag 3 is inflated through the second inflatable connection tube 4, and the elastic bag 3 is tightly close to the intestinal wall forming a second section sealing; and the first section sealing and the second section sealing is to prevent the waste matter in the intestine from leaking along the intestinal wall. The annular superabsorbent material 8 is disposed at an outlet of the ostomy outside the abdominal wall, which can absorb the waste matter in the intestine leaking slowly. The ostomy outside the abdominal wall is to suture the everted intestine at the abdominal wall, the first sealing ring 2 and the turning horn mouth 12 are turned outward toward the abdomina wall in the direction of the everted intestine, and the ostomy exposed outside the abdominal wall are covered, thereby protecting the exposed ostomy; and the multi-cavity inflatable bag 5 is inflated through the first inflatable connection tube 6, and four bags (5-1, 5-2, 5-3, 5-4) expand inward to close the cavity of the ostomy connection tube 1. In this way, the discharge of the waste matter in the intestine can be controlled, such that the patient the ostomy can regularly discharge the waste matter.

It should be noted that the second sealing ring 7 is disposed at the turning outward line. As one embodiment, the second sealing ring 7 is taken as the turning outward line, the turning horn mouth 12 with the first sealing ring 2 is turned outward, and two layers of the elastic material is sealed together to form the elastic bag 3 by a welding process or a heat sealing process in the position aligned with or close to the multi-cavity inflatable bag 5. As another embodiment, the second sealing ring 7 is not disposed, this position is taken as the turning outward line, the turning horn mouth 12 with the first sealing ring 2 is turned outward, and two layers of the elastic material is sealed together to form the elastic bag 3 by the welding process or the heat sealing process in the position aligned with or close to the multi-cavity inflatable bag 5.

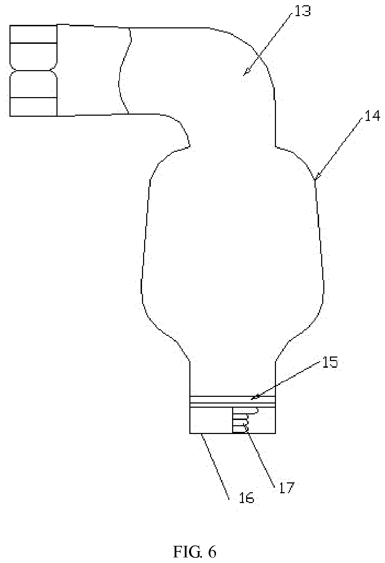

As shown in FIG. 6, the ostomy waste matter collection device also includes an extension tube connected with the ostomy connection tube; and the person with the ostomy can put the extension tube in the waste matter collector. As one embodiment, the extension tube is connected with a collection pocket, and one end of the collection pocket is provided with a discharge controlling assembly. The discharge controlling assembly includes a sealing pocket 16 extended from the collection pocket, a self-sealing strip 15 fixed at the sealing pocket 16, a soft magnetic strip disposed at a lower end of the self-sealing strip 15, and a handle 17 fixed at the sealing pocket 16 and located at a lower end of the soft magnetic strip. The soft magnetic strip is designed to facilitate aligning the self-sealing strip 15 with the uneven position, which facilitates sealing and improves the sealing property; and the handle 17 is disposed to help open the sealing pocket 16 and the self-sealing strip 15, thereby discharging the waste matter. This design makes it easy to discharge the waste matter and reuse it. When the person with the ostomy is unwilling to defecate regularly or it is inconvenience to defecate, the multi-cavity inflatable bag 5 can be inflated to temporarily leave the waste matter in the collection pocket 14.

This invention provides the ostomy waste matter collection device. The second sealing ring 7 and the sealing member cooperate to improve the sealing property, thereby avoiding the leakage of the waste matter; by disposing the discharge valve, the discharge of the waste matter in the intestine can be controlled, such that the patient with the ostomy can regularly discharge the waste matter; by disposing the ostomy protection assembly, the waste matter in the intestine can be effectively controlled and prevented from leaking to the skin surrounding the ostomy, thereby avoiding ulceration of the skin and ensuring health of the patient.

## Claims

1. An ostomy waste matter collection device comprising an ostomy connection tube (1) adapted to be fixed in an ostomy, a discharge valve disposed in the ostomy connection tube (1) and used for controlling discharge of a waste matter, a sealing member disposed between the ostomy connection tube (1) and an abdominal wall (10), in use, for sealing, a turning outward line disposed between the discharge valve and the sealing member, and an ostomy protection assembly for covering the ostomy exposed outside the abdominal wall (10) and being connected with the ostomy connection tube (1),
**characterised in that** the discharge valve comprises a multi-cavity inflatable bag (5) disposed in the ostomy connection tube (1) and a first inflatable connection tube (6) connected with the multi-cavity inflatable bag (5), the multi-cavity inflatable bag (5) expandable inwardly to close a cavity of the ostomy connection tube (1) when the multi-cavity inflatable bag (5) is inflated;
wherein the sealing member comprises an elastic bag (3) disposed at the ostomy connection tube (1) and a second inflatable connection tube (4) connected with the elastic bag (3);
wherein the ostomy protection assembly comprises a turning horn mouth (12) extended from the ostomy connection tube (1) and used for covering an ostomy exposed portion (9), and a first sealing ring (2) disposed at an end of the turning horn mouth (12);
wherein a second sealing ring (7) fixed in the ostomy, in use, is disposed at the turning outward line;
wherein the turning outward line is a center line when the turning horn mouth (12) with the first sealing ring (2) is turned outward till the elastic bag (3) is aligned with or close to the multi-cavity inflatable bag (5);
wherein the second sealing ring (7) is adapted to open an intestinal wall and be tightly close to the abdominal wall (10) after the second sealing ring (7) enters the ostomy, and the second sealing ring (7) and the elastic ostomy connection tube (1) adapted to cooperate to form a first section sealing; the elastic bag (3) being inflatable through the second inflatable connection tube (4), and the elastic bag (3) is adapted to be tightly close to the intestinal wall to form a second section sealing; and the first section sealing and the second section being adapted to sealingly cooperate to prevent the waste matter in the intestine from leaking along the intestinal wall.

2. The ostomy waste matter collection device according to claim 1, wherein the elastic bag (3) is coated with a lubricating and sealing reagent.

3. The ostomy waste matter collection device according to claim 1, wherein an absorbent material is disposed between the turning horn mouth (12) and the ostomy exposed portion (9).

4. The ostomy waste matter collection device according to claim 1, further comprising an extension tube (13) connected with the ostomy connection tube (1).

5. The ostomy waste matter collection device according to claim 4, further comprising a collection pocket (14) connected with the extension tube (13), wherein one end of the collection pocket (14) is provided with a discharge controlling assembly.

6. The ostomy waste matter collection device according to claim 5, wherein the discharge controlling assembly comprises: a sealing pocket (16) extended from the collection pocket (14), a self-sealing strip (15) fixed at the sealing pocket (16), a soft magnetic strip disposed at a lower end of the self-sealing strip (15), and a handle (17) fixed at the sealing pocket (16) and located at a lower end of the soft magnetic strip (15).

## Patentansprüche

1. Vorrichtung zum Sammeln von Stomaausscheidungen umfassend einen Stomaverbindungsschlauch (1), der dafür geeignet ist, um in einem Stoma befestigt zu werden, ein Ausflussventil, das in dem Stomaverbindungsschlauch (1) angeordneten ist, und zum Steuern des Ausflusses von Ausscheidungen verwendeten wird, ein Abdichtungselement, das zwischen dem Stomaverbindungsschlauch (1) und einer Bauchwand (10) in Verwendung zum Abdichten einer sich nach außen biegenden Leitung angeordnet ist, die zwischen dem Ausflussventil und dem Abdichtungselement angeordnet ist, und eine Stomaschutzanordnung zum Abdecken des Stomas, das außerhalb der Bauchwand (10) freigelegt und mit dem Stomaverbindungsschlauch (1) verbunden ist, **dadurch gekennzeichnet, dass**
das Ausflussventil einen aufblasbaren Multi-Hohlraumbeutel (5), der in dem Stomaverbindungsschlauch (1) angeordnet ist, und einen ersten aufblasbaren Verbindungsschlauch (6), der mit dem aufblasbaren Multi-Hohlraumbeutel (5) verbunden ist, umfasst, wobei der aufblasbare Multi-Hohlraumbeutel (5) nach innen expandierbar ist, um einen Hohlraum des Stomaverbindungsschlauchs (1) zu schließen, wenn der aufblasbare Multi-Hohlraumbeutel (5) aufgeblasen ist;
wobei das Abdichtungselement einen elastischen Beutel (3), der an dem Stomaverbindungsschlauch (1) angeordnet ist, und einen zweiten aufblasbaren Verbindungsschlauch (4), der mit dem elastischen Beutel (3) verbunden ist, umfasst;
wobei die Stomaschutzanordnung eine drehbare Hornmündung (12), die sich von dem Stomaverbindungsschlauch (1) erstreckt und zum Abdecken eines freigelegten Stomaabschnitts (9) verwendet wird, und einen ersten Abdichtungsring (2), der an einem Ende der drehbaren Hornmündung (12) angeordnet ist, umfasst;
wobei ein zweiter Abdichtungsring (7), der in dem Stoma befestigt ist, in Verwendung an einer sich nach außen biegenden Leitung angeordnet ist:
wobei die sich nach außen biegende Leitung eine mittlere Leitung ist, wenn die sich drehende Hornmündung (12) mit dem ersten Abdichtungsring (2) nach außen hin gedreht wird bis der elastische Beutel (3) mit dem aufblasbaren Multi-Hohlraumbeutel (5) ausgerichtet oder nahe daran ist;
wobei der zweite Abdichtungsring (7) dafür geeignet ist, um eine Darmwand zu öffnen und an der Bauchwand (10) eng anzuliegen, nachdem der zweite Abdichtungsring (7) in das Stoma eintritt, und der zweite Abdichtungsring (7) und der elastische Stomaverbindungsschlauch (1) dafür geeignet sind, um zusammenzuwirken, um einen ersten Abdichtungsbereich auszubilden;
wobei der elastische Beutel (3) durch den zweiten aufblasbaren Verbindungsschlauch (4) aufblasbar ist, und der elastische Beutel (3) dafür geeignet ist, um an der Darmwand eng anzuliegen, um einen zweiten Abdichtungsbereich auszubilden; und
wobei der erste Abdichtungsbereich und der zweite Abdichtungsbereich dafür geeignet sind, um abdichtend zusammenzuwirken, um zu verhindern, dass Abscheidungen in dem Darm entlang der Darmwand austreten.

2. Vorrichtung zum Sammeln von Stomaausscheidungen nach Anspruch 1, wobei der elastische Beutel (3) mit einem Schmier- und Abdichtreagens beschichtet ist.

3. Vorrichtung zum Sammeln von Stomaausscheidungen nach Anspruch 1, wobei ein absorbierendes Material zwischen der sich drehenden Hornmündung (12) und dem freigelegten Stomaabschnitt (9) angeordnet ist.

4. Vorrichtung zum Sammeln von Stomaausscheidungen nach Anspruch 1, ferner umfassend einen Verlängerungsschlauch (13), der mit dem Stomaverbindungsschlauch (1) verbunden ist.

5. Vorrichtung zum Sammeln von Stomaausscheidungen nach Anspruch 4, ferner umfassend eine Sammeltasche (14), die mit dem Verlängerungsschlauch (13) verbunden ist, wobei ein Ende der Sammeltasche (14) mit einer Ausflusssteuerungsanordnung versehen ist.

6. Vorrichtung zum Sammeln von Stomaausscheidungen nach Anspruch 5, wobei die Ausflusssteuerungsanordnung umfasst:
eine Abdichtungstasche (16), die sich von der Sammeltasche (14) erstreckt, einen selbstabdichtenden Streifen (15), der an der Abdichtungstasche (16) befestigt ist, einen weichmagnetischen Streifen, der an einem unteren Ende des selbstabdichtenden Streifens (15) angeordnet ist und einen Griff (17), der an der Abdichtungstasche (16) befestigt ist und sich an einem unteren Ende des weichmagnetischen Streifens (15) befindet.

## Revendications

1. Dispositif de collecte d'excréments de stomie comprenant un tube de raccordement de stomie (1) adapté pour être fixé dans une stomie, une valve d'évacuation disposée dans le tube de raccordement de stomie (1) et utilisée pour réguler l'évacuation d'excréments, un élément d'étanchéité disposé entre le tube de raccordement de stomie (1) et une paroi abdominale (10), en utilisation, pour assurer l'étanchéité, une ligne tournant vers l'extérieur disposée entre la valve d'évacuation et l'élément d'étanchéité, et un ensemble de protection de stomie pour envelopper la stomie exposée à l'extérieur de la paroi abdominale (10) et étant raccordé au tube de raccordement de stomie (1), **caractérisé en ce que**
la valve d'évacuation comprend un sac gonflable multi-cavités (5) disposé dans le tube de raccordement de stomie (1) et un premier tube de raccordement gonflable (6) raccordé au sac gonflable multi-cavités (5), le sac gonflable multi-cavités (5) extensible vers l'intérieur pour fermer une cavité du tube de raccordement de stomie (1) lorsque le sac gonflable multi-cavités (5) est gonflé ;
dans lequel l'élément d'étanchéité comprend un sac élastique (3) disposé au niveau du tube de raccordement de stomie (1) et un second tube de raccordement gonflable (4) raccordé au sac élastique (3) ;
dans lequel l'ensemble de protection de stomie comprend une bouche de corne tournante (12) s'étendant depuis le tube de raccordement de stomie (1) et utilisée pour couvrir une partie exposée de stomie (9), et une première bague d'étanchéité (2) disposée à une extrémité de la bouche de corne tournante (12) ;
dans lequel une seconde bague d'étanchéité (7) fixée dans la stomie, en utilisation, est disposée au niveau de la ligne tournant vers l'extérieur :
dans lequel la ligne tournant vers l'extérieur est une ligne médiane lorsque la bouche de corne tournante (12) avec la première bague d'étanchéité (2) est tournée vers l'extérieur jusqu'à ce que le sac élastique (3) soit aligné avec ou proche du sac gonflable multi-cavités (5) ;
dans lequel la seconde bague d'étanchéité (7) est adaptée pour ouvrir une paroi intestinale et être étroitement proche de la paroi abdominale (10) après que la seconde bague d'étanchéité (7) entre dans la stomie, et la seconde bague d'étanchéité (7) et le tube de raccordement de stomie (1) sont adaptés pour coopérer pour former une première section d'étanchéité ;
le sac élastique (3) étant gonflable à travers le second tube de raccordement gonflable (4), et le sac élastique (3) est adapté pour être étroitement proche de la paroi intestinale pour former une seconde section d'étanchéité ; et
la première section d'étanchéité et la seconde section étant adaptées pour coopérer de manière étanche afin d'éviter la fuite des excréments dans l'intestin le long de la paroi intestinale.

2. Dispositif de collecte d'excréments de stomie selon la revendication 1, dans lequel le sac élastique (3) est enduit d'un réactif de lubrification et d'étanchéité.

3. Dispositif de collecte d'excréments de stomie selon la revendication 1, dans lequel un matériau absorbant est disposé entre la bouche de corne tournante (12) et la partie exposée de stomie (9).

4. Dispositif de collecte d'excréments de stomie selon la revendication 1, comprenant en outre un tube d'extension (13) raccordé au tube de raccordement de stomie (1).

5. Dispositif de collecte d'excréments de stomie selon la revendication 4, comprenant en outre une poche de collecte (14) raccordée au tube d'extension (13), dans lequel une extrémité de la poche de collecte (14) est pourvue d'un ensemble de régulation d'évacuation.

6. Dispositif de collecte d'excréments de stomie selon la revendication 5, dans lequel l'ensemble de régulation d'évacuation comprend :
une poche d'étanchéité (16) s'étendant depuis la poche de collecte (14), une bande auto-obturante (15) fixée au niveau de la poche d'étanchéité (16), une bande magnétique souple disposée à une extrémité inférieure de la bande auto-obturante (15), et une poignée (17) fixée au niveau de la poche d'étanchéité (16) et située à une extrémité inférieure de la bande magnétique souple (15).
